# EUROPEAN PATENT SPECIFICATION

(11) **EP 1 519 728 B1**
(45) Date of publication and mention of the grant of the patent: **11.03.2009**
(21) Application number: 03762820.3
(22) Date of filing: 09.07.2003
(51) Int. Cl.: A61K 31/519, A61P 1/00

(54) **4-(2-FLUOROPHENYL)-6-METHYL-2(1-PIPERAZINYL)THIENO(2,3-D) PYRIMIDINE IN THE TREATMENT OF FUNCTIONAL BOWEL DISORDER**
4-(2-FLUOROPHENYL)-6-METHYL-2(1-PIPERAZINYL)THIENO(2,3-D) PYRIMIDIN BEI DER BEHANDLUNG VON FUNKTIONALEN DARMSTÖRUNGEN
4-(2-FLUOROPHENYL)-6-METHYL-2(1-PIPERAZINYL)THIENO(2,3-D) PYRIMIDINE DESTINEE A TRAITER UN TROUBLE INTESTINAL FONCTIONNEL

(30) Priority: 10.07.2002 GB 0216027; 28.02.2003 GB 0304648
(43) Date of publication of application: 06.04.2005
(73) Proprietor: Dynogen Pharmaceuticals, Inc., Waltham, MA 02451 (US)
(72) Inventor: CAVALLA, David, Cambridge CB4 0WS (GB); GRISTWOOD, Robert, William, Cambridge CB4 0WS (GB)
(74) Representative: Snodin, Michael D.
(86) International application number: PCT/GB2003/002974
(87) International publication number: WO 2004/004734

(56) References cited:
- DE-A- 10 063 223
- US-A- 4 695 568
- US-B1- 6 284 770
- EGUCHI J ET AL: "The anxiolytic-like effect of MCI-225, a selective NA reuptake inhibitor with 5-HT3 receptor antagonism" PHARMACOLOGY BIOCHEMISTRY AND BEHAVIOR, ELSEVIER, US, vol. 68, no. 4, April 2001 (2001-04), pages 677-683, XP002239887 ISSN: 0091-3057
- CROWELL M D: "The role of serotonin in the pathophysiology of irritable bowel syndrome" AMERICAN JOURNAL OF MANAGED CARE 2001 UNITED STATES, vol. 7, no. 8 SUPPL., 2001, pages S252-S260, XP009018426 ISSN: 1088-0224

## Description

### Field of the Invention

This invention relates to a new use of 4-(2-fluorophenyl)-6-methyl-2-(1-piperazinyl)thieno(2,3-d)pyrimidine in the treatment of functional bowel disorder.

### Background of the Invention

4-(2-Fluorophenyl)-6-methyl-2-(1-piperazinyl)thieno[2,3-D]pyrimidine monohydrate hydrochloride is known (see US-A-4695568) and has shown activity as an antidepressant. It has serotonin and noradrenergicreuptake blocking properties and this is thought to be the mechanism for its action as an antidepressant. The compound also has 5HT-3 blocking activity.

Functional bowel disorders are very common and include irritable bowel syndrome (IBS) and functional dyspepsia. IBS is the most common disorder diagnosed by gastroenterologists and one of the more common encountered in general practice. The overall prevalence rate is similar (approx 10%) in most industrialised countries. Some estimates of prevalence have reached 20%. The illness has a large economic impact on health care use and indirect costs, chiefly through absenteeism.

IBS falls into two categories of equal prevalence, constipation-predominant and diarrhoea-predominant. The available treatments are generally poor.

A recent approach to treating diarrhoea-predominant IBS has involved the use of alosetron. This drug works by blocking the 5HT-3 receptor. Other drugs with this mechanism of action have shown some limited activity in this disease, including granisetron. Alosetron, although effective, was withdrawn due to side-effects on the colon.

A recent approach to treating constipation-predominant IBS involved agonising the 5HT4 receptor. Two such agonists are in clinical trials, i.e. tegaserod and prucalopride. Other approaches being explored include using 5HT1 agonists such as buspirone.

Functional dyspepsia is characterised by impaired accommodation of the stomach to a meal and epigastric pain discomfort or pain. There is often early satiety and weight loss. The disorder is not well understood. Treatments include antispasmodics and drugs affecting gut motility. Early studies suggest that buspirone and serotonin reuptake inhibitors may be useful.

### Summary of the Invention

Surprisingly, it has been found that the known compound identified above (referred to herein as MCI-225) has activity in the treatment of functional bowel disorders. Its combination of serotonin and noradrenergic reuptake blockade and 5HT-3 receptor blockade has not previously been clearly identified as being responsible for activity in functional bowel disorders. Furthermore MCI-225, at doses effective in the treatment of bowel disorders, can produce a lower incidence of some of the side-effects which are commonly known to be associated with the clinical use of selective serotonin reuptake inhibitors, for example the production of nausea and vomiting or the induction of sexual dysfunction. It will be appreciated that any suitable form of the active principle may be used, e.g. another salt form, or a prodrug or active metabolite.

### Description of Preferred Embodiments

By means of this invention, functional bowel disorders and associated pain symptoms can be treated, e.g. controlled or prevented. Such disorders include irritable bowel syndrome, including diarrhoea-predominant, constipation-predominant, and alternating constipation/diarrhoea IBS. The patient may be male or female, diarrhoea-predominant IBS being particularly associated with women.

For use in the invention, the active compound can be formulated in any suitable manner together with a conventional diluent or carrier. The active compound is preferably administered by the oral route; other suitable routes of administration include sublingual/buccal, transdermal, intramuscular, intranasal, rectal, parenteral, subcutaneous, pulmonary and topical. An effective dose of the active agent will depend on the nature and degree of the complaint, the age and condition of the patient and other factors known to those skilled in the art. A typical daily dosage may be 0.1 mg to 1 g.

A pharmaceutical composition containing the active ingredient may be in the form of a sublingual tablet or patch. Suitable compositions for oral use include tablets, troches, lozenges, aqueous or oily suspensions, dispersible powders or granules, emulsions, hard or soft capsules, syrups and elixirs. Suitable additives include sweetening agents, flavouring agents, colouring agents and preserving agents. Tablets contain the active ingredient in admixture with non-toxic pharmaceutically acceptable excipients, e.g. inert diluents such as calcium carbonate, sodium carbonate, lactose, calcium phosphate or sodium phosphate; granulating and disintegrating agents, for example corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastointestinal tract and thereby provide a sustained action over a longer period. For example, a time delay material such as glyceryl monostearate or glyceryl distearate may be employed. They may also be coated, to form osmotic therapeutic tablets for controlled release. Hard gelatin capsules may include an inert solid diluent, for example calcium carbonate, calcium phosphate or kaolin; soft gelatin capsules may include water or an oil medium, for example peanut oil, liquid paraffin or olive oil.

The data on which this invention is based will now be described. In a study using intact animals, the ability of a drug to inhibit the reflex depressor response to colorectal distension can be assessed. In this model, an inhibition of the reflex indicates modulation of visceral nociceptive neurotransmission and, therefore, the use of the drug in functional bowel disease (e.g. IBS); see Kozlowski et al, 2000, Gut 46, 474-480. Allodynia and visceral pain are important components of functional bowel disease.

### Study

Experiments were performed on male Sprague-Dawley rats (250-300 g). Anaesthesia was induced with isoflurane (2.5% in oxygen) and maintained with alpha chlorolose (80 mg/kg i.v.). The left carotid artery was cannulated for the measurement of blood pressure and heart rate and the left jugular vein cannulated for drug administration. A tracheal cannula was implanted for artificial respiration if required. A 10 mm long latex balloon was inserted intrarectally so that the tip of the balloon was 20 mm from the anal verge (Kozlowski *et al*, *supra*). The balloon was connected via a double lumen cannula to a pressure transducer and also to a saline-filled syringe for inflation/deflation of the balloon. Throughout the experiment, body temperature was kept constant at 36-38 C using a homeothermic blanket.

Once stable baseline parameters were obtained (approximately after 20 minutes), the balloon was rapidly inflated with increasing volumes of saline (0.5-2.5 ml) for 30 seconds at 5 minute intervals, and the resultant change in blood pressure recorded. Three distinct response curves were constructed, with a 10 minute stabilisation period between each curve. In one group of animals, 10 minutes prior to the commencement of the final distension response curve, a single bolus of MCI-225 (3 mg/kg) was administered intravenously; in a second group of animals, a single bolus dose of vehicle was administered. The effect of MCI-225 and vehicle was determined by analysing the changes in colorectal distension that evoked depressor response.

Falls in arterial blood pressure (mean absolute decreases in mean arterial pressure in mmHg, with standard error of mean in brackets) evoked by distension of the balloon, before adding drug, at 0.5, 1.0, 1.5, 2.0 and 2.5 ml balloon volume were 2.7 (1.9), 12.4 (5.9), 24.0 (8.9), 36.3 (4.8) and 43.4 (6.0), respectively (all except final value n=6, final value n=5). Following administration of MCI-225 at 3 mg/kg i.v., the corresponding values were 2.2 (1.65), 6.3 (2.6), 10.6 (3.9), 15.3 (5.4) and 24.6 (7.3), respectively (all values except final value n=6, final value n=5).

The results clearly show that MCI-225 inhibited the distension-induced falls in blood pressure. The falls in blood pressure evoked by 2.0 and 2.5 ml balloon volumes were reduced with statistical significance following administration of MCI-225 at 3mg/kg, with p values (paired t test) of less than 0.01 and less than 0.05 respectively.

## Claims

1. Use of (4-(2-fluorophenyl)-6-methyl-2-(1-piperazinyl)thieno[2,3-D]pyrimidine or a salt thereof for the manufacture of a medicament for the treatment of a functional bowel disorder.

2. Use according to claim 1, wherein the salt is the hydrochloride monohydrate.

3. Use according to claim 1 or claim 2, wherein the disorder is irritable bowel syndrome.

4. Use according to claim 3, wherein the disorder is diarrhoea-predominant irritable bowel syndrome.

5. Use according to claim 4, wherein the disorder is in a female patient.

6. Use according to claim 3, wherein the disorder is alternating constipation/diarrhoea irritable bowel syndrome.

7. Use according to claim 3, wherein the disorder is constipation-predominant irritable bowel syndrome.

## Patentansprüche

1. Verwendung von 4-(2-Fluorphenyl)-6-methyl-2-(1-piperazinyl)thieno[2,3-D]pyrimidin oder einem Salz hiervon zur Herstellung eines Medikaments zur Behandlung einer funktionellen Darmstörung.

2. Verwendung nach Anspruch 1, wobei das Salz das Hydrochloridmonohydrat ist.

3. Verwendung nach Anspruch 1 oder Anspruch 2, wobei die Störung Reizdarmsyndrom ist.

4. Verwendung nach Anspruch 3, wobei die Störung Diarrhoe-dominiertes Reizdarmsyndrom ist.

5. Verwendung nach Anspruch 4, wobei die Störung bei einem weiblichen Patienten vorliegt.

6. Verwendung nach Anspruch 3, wobei die Störung Reizdarmsyndrom mit abwechselnd Konstipation/Diarrhoe ist.

7. Verwendung nach Anspruch 3, wobei die Störung Konstipation-dominiertes Reizdarmsyndrom ist.

## Revendications

1. Utilisation de (4-(2-fluorophényl)-6-méthyl-2-(1-pipérazinyl)thiéno[2,3-D]pyrimidine ou d'un sel de celle-ci pour la préparation d'un médicament destiné au traitement d'un trouble intestinal fonctionnel.

2. Utilisation selon la revendication 1, dans laquelle le sel est le chlorhydrate monohydraté.

3. Utilisation selon la revendication 1 ou la revendication 2, dans laquelle le trouble est le syndrome du côlon irritable.

4. Utilisation selon la revendication 3, dans laquelle le trouble est le syndrome du côlon irritable à prédominance diarrhéique.

5. Utilisation selon la revendication 4, dans laquelle le trouble se situe chez une patiente.

6. Utilisation selon la revendication 3, dans laquelle le trouble est le syndrome du côlon irritable avec une alternance constipation/diarrhée.

7. Utilisation selon la revendication 3, dans laquelle le trouble est le syndrome du côlon irritable à prédominance de constipation.
